# EUROPEAN PATENT SPECIFICATION

(11) **EP 1 365 261 B1**
(45) Date of publication and mention of the grant of the patent: **14.12.2016**
(21) Application number: 02710408.2
(22) Date of filing: 30.01.2002
(51) Int. Cl.: G01T 1/20, G21K 4/00

(54) **SCINTILLATOR PANEL AND RADIATION IMAGE SENSOR**
SZINTILLATORTAFEL UND STRAHLUNGSBILDSENSOR
PANNEAU DE SCINTILLATION ET DETECTEUR D'IMAGES DE RAYONNEMENT

(30) Priority: 30.01.2001 JP 2001022167; 05.11.2001 JP 2001339741
(43) Date of publication of application: 26.11.2003
(73) Proprietor: HAMAMATSU PHOTONICS K. K., Hamamatsu-shi, Shizuoka 435-8558 (JP)
(72) Inventor: SATO, Hiroto, c/o Hamamatsu Photonics K.K., Hamamatsu-shi, Shizuoka 435-8558 (JP); TAKABAYASHI, Toshio, c/o Hamamatsu Photonics K.K., Hamamatsu-shi, Shizuoka 435-8558 (JP); SUZUKI, Takaharu, c/o Hamamatsu Photonics K.K., Hamamatsu-shi, Shizuoka 435-8558 (JP); NATSUME, Yoshio, c/o Hamamatsu Photonics K.K., Hamamatsu-shi, Shizuoka 435-8558 (JP)
(74) Representative: Boult Wade Tennant
(86) International application number: PCT/JP2002/000732
(87) International publication number: WO 2002/061459

(56) References cited:
- EP-A1- 1 326 093
- EP-A2- 0 327 134
- WO-A1-99/66350
- WO-A1-99/66351
- WO-A1-99/66352
- AU-A- 1 507 399
- JP-A- 56 089 702
- JP-A- 61 073 901
- JP-A- 2000 356 679
- JP-A- 2000 356 679
- US-A- 453 485
- US-A- 5 179 284

## Description

### Technical Field

The present invention relates to a scintillator panel and radiation image sensor which are used for medical X-ray photography or the like.

### Background Art

While X-ray sensitive films have conventionally been used in medical and industrial X-ray photography, radiation imaging systems using radiation detectors have been coming into widespread use from the viewpoint of convenience and storability of photographed results. In such a radiation imaging system, pixel data caused by two-dimensional radiation are acquired as an electric signal by a radiation detector, and this signal is processed by a processing unit, so as to be displayed on a monitor.

Conventionally known as a typical radiation detector is one having a structure in which a scintillator panel comprising a scintillator formed on a substrate made of aluminum, glass, fused silica, or the like and an imaging device are cemented together. In this radiation detector, the radiation entering from the substrate side is converted by the scintillator into visible light, which is then detected by the imaging device (see JP7-21560B).

US-A-5,179,284 discloses a radiation imager having a scintillator formed upon a photodetector array. The light incident surface of the scintillator, distal from the photodetector array, is provided with a pellicle layer, and a moisture barrier is formed on top of that. The moisture barrier includes an optically reflective, radiation transmissive metal layer and a moisture sealant layer. A radiation transmissive window is disposed on top of the moisture barrier.

### Disclosure of the Invention

Meanwhile, though it is necessary for the scintillator panel to have a sufficiently high optical output in order to attain clear images in a radiation detector, the optical output has not been sufficient in the above-mentioned radiation detector. Therefore, the inventors of the present application developed a technique by further covering a reflecting metal thin film disposed on a substrate with a protective film which prevents degradation etc. from the moisture slightly included in the scintillator and prevents deterioration of the function of the metal thin film as the reflecting film as disclosed in JP2000-356679A.

It is an object of the present invention to supply a method of manufacture of a scintillator panel further enhancing the stability of a metal thin film as a reflecting film and a radiation image sensor using this scintillator panel.

To achieve above-mentioned object, a method of manufacture of a scintillator panel according to the present invention is defined in claim 1.

In some combinations of the substrate and the metal thin film, the adhesiveness of the metal thin film to the substrate is not favourable, there is a possibility that the thin film peels off from the substrate upon the manufacturing process or under usage thereafter. By disposing the intermediate film between the metal thin film and the substrate which adheres both, it prevents the peeling off of the metal thin film and enhances the stability of reflecting film.

It is preferable that the substrate is an aluminum substrate or a substrate predominantly composed of carbon. Especially, when using a substrate predominantly composed of carbon, such as amorphous carbon or graphite based substrate, an adhesiveness of a metal thin film to the substrate is poor, however, according to the present invention, through the mediation of the intermediate film the adhesiveness can be enhanced.

When the conductive substrate contacts the metal thin film the metal thin film may rust by galvanic corrosion. According to the present invention, the non-conductive intermediate film prevents the contact between the metal thin film and the conductive substrate, and it can suppress such galvanic corrosion and enhance the stability of metal thin film.

The reflective film may be substantially made of a material containing a substance selected from the group consisting of Al, Ag, Cr, Cu, Ni, Ti, Mg, Rh, Pt and Au.

The protective film may be an inorganic film, organic film or combination thereof. The inorganic film may be substantially made of a material containing a substance selected for the group consisting of LiF, MgF₂, SiO₂, TiO₂, Al₂O₃, MgO, SiN, or the metal oxide film may be an oxidized material containing a substance selected from the group consisting of Al, Ag, Cr, Cu, Ni, Ti, Mg, Rh, Pt and Au. The organic film may be a film containing a substance selected from the group consisting of polyimide or xylylene based material.

It is preferable that the scintillator is covered with an organic film because the water-vapor resistance can be improved. The organic film further reaches at least to the part of the substrate surface, more preferably substantially covers the whole surface of the substrate for achieving an accurate sealing of the scintillator by the organic film.

Above-mentioned intermediate film may be an inorganic film, organic film or combination thereof. For example, the organic film may be polyimide, epoxy based resin or acrylic based resin etc. and the inorganic film may be SiO₂ or SiN etc.

Also, the metal film may adhere the intermediate film and the protective film and may be sealed with both for enhancing its stability. Further, it is preferable that the intermediate film encapsulates the substrate because its sealing property can be enhanced.

This intermediate film may be film formed on the substrate by CVD process, and especially may be xylylene based film.

### Brief Description of the Drawings

Fig. 1 is a sectional view of a scintillator panel manufactured according to the first embodiment, and Fig. 2 is a sectional view of a radiation image sensor manufactured according to the first embodiment;
Fig. 3 is a sectional view of a scintillator panel manufactured according to a second embodiment; and
Fig. 4 is a sectional view of a scintillator panel manufactured according to the third embodiment.

### Best Modes for Carrying Out the Invention

We will describe some preferred embodiments of the present invention hereinafter. To facilitate the comprehension of the explanation, the same reference numerals denote the same parts, where possible, throughout the drawings, and a repeated explanation will be omitted. Incidentally, the dimensions in the figures are slightly exaggerated for purposes of illustration, and do not always match actual dimensions.

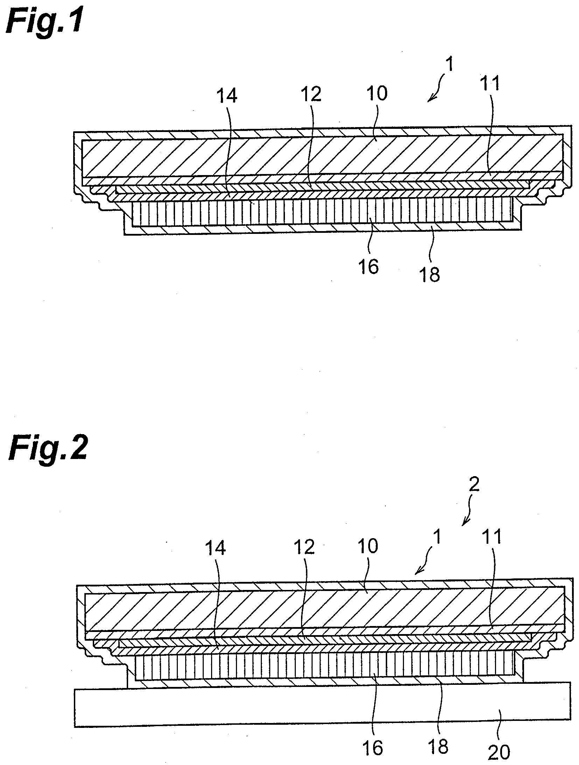

The first embodiment of the present invention will be described below with reference to Figs. 1 and 2. Fig. 1 is a sectional view of a scintillator panel 1, and Fig. 2 is a sectional view of a radiation image sensor 2.

As shown in Fig. 1, a polyimide intermediate film 11 is disposed on one surface of the amorphous carbon (a-C) (glassy carbon or glass-like carbon) substrate 10 of the scintillator panel 1 by adhesion, and a metal thin film 12 which functions as a light-reflecting film is formed on the surface of this intermediate film 11 by adhesion. This metal thin film is manufactured from Al, for example. The surface of this metal thin film 12 is covered by a polyimide protective film 14 for protecting the metal thin film 12. As a result, the metal thin film 12 is sandwiched between, and thus sealed by, the intermediate film 11 and the protective film 14 to which it is adhered. A scintillator 16 with a columnar structure, which converts incident radiation into visible light, is formed on the surface of the protective film 14. In other words, the structure of the scintillator 16 is such that a large number of columnar crystals stand grouped together on the protective film 14. Further, a Tl-doped CsI is used in the scintillator 16. This scintillator 16, as well as the substrate 10, is covered with a polyparaxylylene moisture-resistant protective film 18.

As is shown in Fig. 2, the radiation image sensor 2 has a configuration in which the light-receiving surface of an image-sensing element 20 is affixed to the side of the scintillator, panel 1 on which the scintillator 16 is formed.

Next, the making process for the scintillator panel 1 will be explained. First, polyimide resin is painted onto one surface of the rectangular or circular a-C substrate 10 (thickness: 1 mm) at a constant thickness (10 µm) and caused to harden, thereby becoming adhered to the substrate 10 and forming the intermediate film 11 with a flat surface.

The metal thin film 12 which functions as a light-reflecting film is formed on the surface of this intermediate film 11 at a thickness of 150nm by vacuum deposition. The polyimide constituting the intermediate film 11 has a good affinity with the Al metal thin film 12, and hence, the metal thin film 12 becomes adhered to the intermediate film 11. Since the intermediate film 11 is also adhered to the substrate 10, peeling away of the metal thin film 12 from the substrate 10 can be effectively prevented.

Subsequently, spin coat processing is applied onto the metal thin film 12, thus forming a polyimide protective film 14 at a thickness of 1000nm which covers the entire metal thin film 12. As a result, the metal thin film 12 is sandwiched between the intermediate film 11 and the protective film 14 so as to be adhered to and sealed by both, and hence canbe effectively protected from damage or peeling in the subsequent making process.

Next, a large number of Tl-doped CsI columnar crystals are grown (accumulated) by deposition on the surface of the protective film 14 so as to stand grouped together, thereby forming the scintillator 16 at a thickness of 250 µm. The CsI that forms the scintillator 16 has high hygroscopicity, and if left exposed, absorbs vapor in the air and deliquesces. In order to prevent this, the polyparaxylylene moisture-resistant protective film 18 is formed by CVD. That is, the substrate 10 on which the scintillator 16 is formed is inserted into a CVD device, and the moisture-resistant protective film 18 is formed at a thickness of 10 µm. The making method for this moisture-resistant protective film 18 is described in detail in International Publication No. WO99/66351. Thereby, the polyparaxylylene moisture-resistant protective film 18 is formed on substantially the entire surface of the scintillator 16 and the substrate 10, or in other words, substantially the entire surface of the substrate that is exposed and does not have a scintillator or the like formed thereon.

The radiation image sensor 2 is made by disposing the light-receiving portion of an image sensing element (CCD) 20 to face the distal end side of the scintillator 16 of the completed scintillator panel 1 and bonding them (see Fig. 2).

According to the radiation image sensor 2 of this embodiment, radiation which enters from the substrate 10 side is converted into light by the scintillator 16 and detected by the image sensing element 20. Since the light-reflecting metal thin film 12 is provided in the scintillator panel 1 comprising the radiation image sensor 2, the amount of light incident on the light-receiving portion of the image-sensing element 20 can be increased, and the image detected by the radiation image sensor 2 can be made clearer. Further, since the metal thin film 12 is adhered to the polyimide intermediate film 11 and the protective film 14, and thus sandwiched between the two to seal the entire film, damage to the function of the metal thin film 12 as a reflecting film due to deterioration such as corrosion, peeling, or other impairments, canbe prevented, and the stability thereof as a reflecting film can be improved.

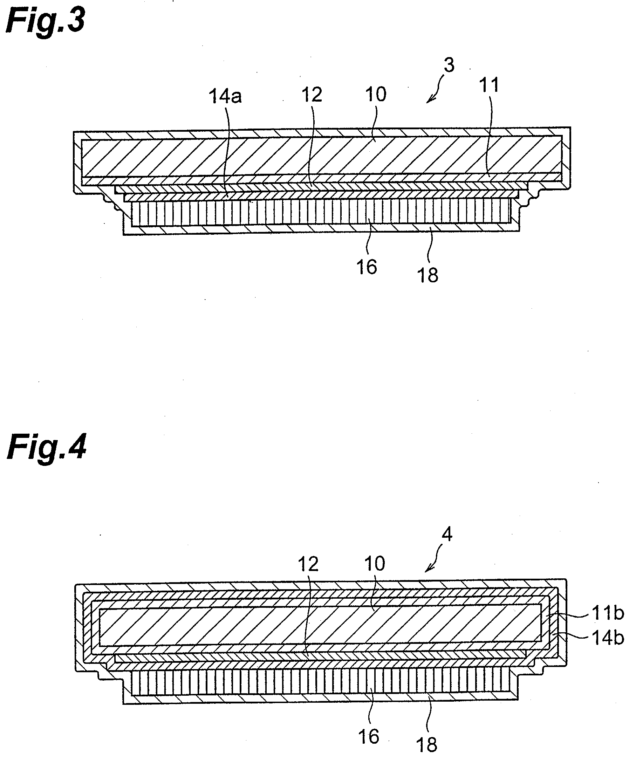

Fig. 3 is a sectional view of a second embodiment of the scintillator panel in accordance with the present invention. This scintillator panel 3 differs from the first embodiment in that the protective film 14a does not cover the entire surface of the metal thin film 12, but rather covers only the central part of the metal thin film 12. In this embodiment also, the scintillator 16 is formed only on the surface of the protective film 14a, and thus the metal thin film 12 is completely prevented from contacting the scintillator 16 by the protective film 34a.

In this embodiment also, at least the scintillator 16 formation part of the metal thin film 12 is sandwiched between, and sealed by, the intermediate film 31 and the protective film 34a, and hence, damage, peeling, deterioration and the like can be effectively prevented, and the stability of the metal thin film 12 as a reflecting film can be improved.

Fig. 4 is a sectional view of a third embodiment of the scintillator panel in accordance with the present invention. This scintillator panel 4 differs from those of the first and second embodiments, illustrated in Figs. 1 and 3, in that a polyparaxylylene film which is the same as the moisture-resistant protective film 18 is used as the intermediate film 11b and the protective film 14b. Furthermore, the intermediate film 11b covers the entire substrate 10, and the protective film 14b covers the metal thin film 12 and the exposed intermediate film 11b on the periphery of the metal thin film 12.

Similarly to the case of the aforementioned moisture-resistant protective film 18 the intermediate film 11b and the protective film 14b are formed by CVD method, thus forming a satisfactory film that is uniform and has no pin holes or the like. As a result the metal thin film 12 is sealed, and contact with the outside air and the scintillator 16 which is formed on the protective film 14b is completely prevented, and thereby, reactions of the metal with scintillator components and moisture can be suppressed. In particular, during the formation of the scintillator 16, the protective film 34b completely covers the substrate 10 and the metal thin film 12, and thus even the effects of the scintillator components becoming attached to another location can be suppressed. Furthermore, by interposing the intermediate film 11b, which is a nonconductor, between the substrate 10 and the metal thin film 12, which have conducting properties, electrical contact between the substrate 10 and the metal thin film 12 can be prevented, and electric corrosion of the metal thin film 12 can be effectively suppressed.

Here, an embodiment was explained in which the intermediate film 11b and the protective film 14b covers the entire substrate 10; however, it is sufficient if the metal thin film 12 is formed on the surface of the intermediate film 11b, and the intermediate film 11b may be formed on only the formation surface side of the scintillator 16 on the substrate. It is also sufficient if the protective film 14b is formed on at least the formation surface part of the scintillator 16, as in the second embodiment. However, if a structure such as that of the present embodiment is employed, wherein the intermediate film 11b and the protective film 14b cover the entire substrate 10, sealing is improved, and the formation of the films by CVD method is simple, and therefore this structure is preferable.

Here, polyparaxylylene film is used, but xylylene based organic film such as polymonochloroparaxylylene, polydichloroparaxylylene, polytetrachloroparaxylylene, polyfluoroparaxylylene, polydimethylparaxylylene, poly-diethylparaxylylene, and the like may be used.

In the above-described embodiments, an a-C substrate is used. However, since the substrate only need to pass radiation, a graphite substrate, A1 substrate, or Be substrate, may be used.

In the above-described embodiments, an oxide film such as polyimide film or xylylene based film is used as a protective film, but the protective film is not limited to this, but may be made of a material containing a substance selected from the group consisting of transparent inorganic films such as LiF, MgF₂, SiO₂, Al₂O₃, TiO₂, MgO, and SiN. Furthermore, a protective film combined with inorganic and organic films may be used.

Also, as a reflective metal thin film, a film made of a material containing a substance selected from the group consisting of Ag, Cr, Cu, Ni, Ti, Mg, Rh, Pt, and Au may be used in addition to Al. In addition, two or more reflective metal thin films may be formed by forming, e.g., an Au film on a Cr film.

Also, it is possible for a protective film to form on a reflective metal thin film its oxide film.

In the above-described embodiments, the entire surfaces of the scintillator 16 and substrate (the surface with the scintillator formed and a surface on the opposite side, i.e., the radiation incident surface) are covered with the moisture-resistant protective film 18, thereby making the scintillator completely resistant against water vapor. When the entire surface of the scintillator 16 and at least part of the surface of the substrate 10 are covered with the moisture-resistant protective film 18, the water-vapor resistance of the scintillator can be made higher than in a case wherein only the scintillator is covered.

Instead of CsI (Tl) as the scintillator 16, CsI (Na), NaI (Tl), LiI (Eu), KI (Tl), or the like may be used.

Furthermore, as the organic film covering the scintillator, xylylen based transparent organic film may be used.

Also, it is necessary for the intermediate film 11 that its material has an affinity for both of the substrate 10 and the metal thin film 12, so in addition to the polyimide or xylylene based transparent organic film, an organic film such as epoxy based resin or acrylic based resin and the like or an inorganic film such as SiO₂ or SiN and the like may be used. For forming the inorganic film, CVD method or firing after painting the inorganic material dispersing in the organic solvent may be used. The substrate is conductive material, and the nonconductive intermediate film is used for preventing the galvanic corrosion by electrical shielding.

In addition, the radiation image sensor according to the present invention is a combination of above-mentioned scintillator panel and image sensing element. The image sensing element may be affixed to the scintillator panel so as to oppose to scintillator 16 as mentioned above, or separated from its surface. Furthermore, the light image is guided by optical system into the image sensing element which is disposed at suitable location.

### Industrial Applicability

The present invention is suitable for large-screen and high-sensitivity X-ray imaging system for medical or industrial use.

## Claims

1. A method of manufacture of a scintillator panel (1), comprising the steps of:
(a) forming a non-conductive intermediate film (11) upon a radiation transmitting conductive substrate (10);
(b) forming a metal thin film (12) upon the non-conductive intermediate film (11), the metal thin film (12) being radiation-transmittable and reflecting light of a predetermined wavelength;
(c) forming a protective film (14) upon the metal thin film (12) and
(d) depositing a scintillator (16) upon the protective film (14), the scintillator comprising a large number of columnar crystals for converting radiation into light of wavelengths that can be reflected by the metal thin film (12); whereby the protective film (14) prevents contact between the metal thin film (12) and the scintillator (16), and further whereby the non-conductive intermediate film (11) adheres to the radiation transmitting, conductive substrate (10) and the metal thin film (12), and prevents contact between said radiation transmitting, conductive substrate (10) and said metal thin film (12).

2. A method according to claim 1, wherein said step (a) of forming the non-conductive intermediate film (11) upon said radiation transmitting conductive substrate (10) comprises forming the non-conductive intermediate film (11) upon either an aluminum substrate or a substrate that has carbon as a main component.

3. A method according to claim 1 or claim 2, wherein said step (a) of forming said non-conductive intermediate film (11) upon said conductive substrate (10) comprises forming the non-conductive intermediate film (11) upon said conductive substrate (10) that contains amorphous carbon.

4. A method according to any one of claims 1 to 3, wherein said step (b) of forming a metal thin film (12) upon the non-conductive intermediate film (11) comprises forming a metal thin film (12) made of a material containing a substance selected from the group consisting of Al, Ag, Cr, Cu, Ni, Ti, Mg, Rh, Pt, and Au, upon the non-conductive intermediate film (11).

5. A method according to any one of claims 1 to 4, wherein said step of forming a protective film (14) upon the metal thin film (12) comprises forming an inorganic film upon the thin metal film (12).

6. A method according to claim 5, wherein said step of forming an inorganic film upon the thin metal film (12) comprises forming an inorganic film, made of material containing a substance selected from the group consisting of LiF, MfF₂, SiO₂, TiO₂, Al₂O₃, MgO, and SiN upon the thin metal film (12).

7. A method according to claim 5, wherein said step of forming an inorganic film upon the thin metal film (12) comprises forming an inorganic film made of material containing a substance selected form the group consisting of Al, Ag, Cr, Cu, Ni, Ti, Mg, Rh, and Pt upon the thin metal film (12).

8. A method according to any one of claims 1 to 4, wherein said step (c) of forming the protective film (14) upon the metal thin film (12) comprises forming an organic film upon the metal thin film (12).

9. A method according to claim 8, wherein said step of forming an organic film upon the metal thin film (12) comprises forming an organic film made of material containing a substance selected from the group consisting of polyimide and xylylene-based materials, upon the metal thin film (12).

10. A method according to any one of claims 1 to 4, wherein said step of forming a protective film (14) upon the metal thin film (12) comprising forming an inorganic film and an organic film upon the metal thin film (12).

11. A method according to any one of claims 1 to 10, further comprising covering said scintillator (16) with an organic film (18).

12. A method according to claim 11, wherein step of covering the scintillator (16) with an organic film (18) comprises extending said organic film (18) to at least one part of the surface of said radiation transmitting, conductive substrate (10).

13. A method according to claim 12, wherein said step of covering the scintillator (16) with an organic film (18) comprises covering substantially the entire surface of said substrate (12) with the organic film (18).

14. A method according to any one of claims 1 to 13, wherein said step of forming the non-conductive intermediate film (11) upon the radiation transmitting, conductive substrate (10) comprises forming either an organic film or an inorganic film, or a combination of the two, upon the radiation transmitting, conductive substrate (10).

15. A method according to any one of claims 1 to 14, wherein said metal thin film (12) is adhered to said non-conductive intermediate film (11) and said protective film (14) and sealed by both.

16. A method according to claim 15, wherein said non-conductive intermediate film (11) substantially envelops said radiation transmitting, conductive substrate (10).

17. A method according to any one of claims 11, 12 or 13, wherein the step of forming the organic film (18) upon the scintillator comprises forming the organic film (18) upon the scintillator (16) using chemical vapour deposition (CVD).

18. A method according to any one of claims 15 to 17, wherein said non-conductive intermediate film (11) is a xylylene-based film.

19. The method according to Claim 1, wherein the step (a) of forming the non-conductive intermediate film (11) on the radiation transmitting, conductive substrate (10) comprises forming an intermediate film containing at least one of polyimide, epoxy based resin, acrylic based resin, SiO₂, or SiN, upon an Al, Be or amorphous carbon substrate;
wherein the step (b) of forming the metal thin film (12) upon the non-conductive intermediate film (11) comprises forming a metal thin film (12) containing a substance selected from the group consisting of Al, Ag, Cr, Cu, Ni, Ti, Mg, Rh, Pt and Au upon the non-conductive intermediate film (11) ;
and further wherein the step (c) of forming the protective film (14) upon the metal thin film (12) comprises forming polyimide upon the metal thin film (12).

20. The method of any proceeding claim, further comprising disposing a light receiving portion of an image sensing element (20) to face an end side of the scintillator (16), so as to form a radiation image sensor.

## Patentansprüche

1. Verfahren eines Herstellens von einem Scintillator-Panel (1) umfassend die Schritte eines:
(a) Ausbildens eines nicht-leitenden Zwischenfilms (11) auf einem Strahlung-transmittierenden leitenden Substrat (10);
(b) Ausbildens eines metallischen Dünnfilms (12) auf dem nicht-leitenden Zwischenfilm (11), wobei der metallische Dünnfilm (12) Strahlungtransmittierend ist und Licht einer vorbestimmten Wellenlänge reflektiert;
(c) Ausbildens eines Schutzfilms (14) auf dem metallischen Dünnfilm (12) und
(d) Anlagern eines Scintillators (16) auf dem Schutzfilm (14), wobei der Scintillator eine große Anzahl von säulenartigen Kristallen umfasst zum Konvertieren von Strahlung in Licht von Wellenlängen, die durch den metallischen Dünnfilm (12) reflektiert werden können; wobei der Schutzfilm (14) einen Kontakt zwischen dem metallischen Dünnfilm (12) und dem Scintillator (16) verhindert und ferner wobei der nicht-leitende Zwischenfilm (11) anhaftet an
dem Strahlung-transmittierenden leitenden Substrat (10) und dem metallischen Dünnfilm (12) und einen Kontakt zwischen dem Strahlung-transmittierenden leitenden Substrat (10) und dem metallischen Dünnfilm (12) verhindert.

2. Verfahren nach Anspruch 1, wobei der Schritt (a) des Ausbildens des nicht-leitenden Zwischenfilms (11) auf dem Strahlung-transmittierenden leitenden Substrat (10) ein Ausbilden des nicht-leitenden Zwischenfilms (11) auf entweder einem Aluminiumsubstrat oder einem Substrat, das Kohlenstoff als eine Hauptkomponente aufweist, umfasst.

3. Verfahren nach Anspruch 1 oder Anspruch 2, wobei der Schritt (a) des Ausbildens des nicht-leitenden Zwischenfilms (11) auf dem leitenden Substrat (10) ein Ausbilden des nicht-leitenden Zwischenfilms (11) auf dem leitenden Substrat (10), das amorphen Kohlenstoff beinhaltet, umfasst.

4. Verfahren nach irgendeinem der Ansprüche 1 bis 3, wobei der Schritt (b) des Ausbildens eines metallischen Dünnfilms (12) auf dem nicht-leitenden Zwischenfilm (11) ein Ausbilden eines metallischen Dünnfilms (12), der aus Material hergestellt ist beinhaltend eine Substanz, die aus der Gruppe ausgewählt ist, die aus Al, Ag, Cr, Cu, Ni, Ti, Mg, Rh, Pt und Au besteht, auf dem nicht-leitenden Zwischenfilm (11) umfasst.

5. Verfahren nach irgendeinem der Ansprüche 1 bis 4, wobei der Schritt des Ausbildens eines Schutzfilms (14) auf dem metallischen Dünnfilm (12) ein Ausbilden eines anorganischen Films auf dem dünnen metallischen Film (12) umfasst.

6. Verfahren nach Anspruch 5, wobei der Schritt des Ausbildens eines anorganischen Films auf dem dünnen metallischen Film (12) ein Ausbilden eines anorganischen Films, der aus Material hergestellt ist beinhaltend eine Substanz, die ausgewählt ist aus der Gruppe, die aus LiF, MfF₂, SiO₂, TiO₂, Al₂O₃, MgO und SiN besteht, auf dem dünnen metallischen Film (12) umfasst.

7. Verfahren nach Anspruch 5, wobei der Schritt des Ausbildens eines anorganischen Films auf dem dünnen metallischen Film (12) ein Ausbilden eines anorganischen Films, der aus Material hergestellt ist beinhaltend eine Substanz, die aus der Gruppe ausgewählt ist, die aus Al, Ag, Cr, Cu, Ni, Ti, Mg, Rh und Pt Au besteht, auf dem dünnen metallischen Film (12) umfasst.

8. Verfahren nach irgendeinem der Ansprüche 1 bis 4, wobei der Schritt (c) des Ausbildens des Schutzfilms (14) auf dem metallischen Dünnfilm (12) ein Ausbilden eines organischen Films auf dem metallischen Dünnfilm (12) umfasst.

9. Verfahren nach Anspruch 8, wobei der Schritt des Ausbildens eines organischen Films auf dem metallischen Dünnfilm (12) ein Ausbilden eines organischen Films, der aus Material hergestellt ist beinhaltend eine Sub-stanz, die aus der Gruppe ausgewählt ist, die aus polyimiden- und Xylylenebasierten Materialien besteht, auf dem metallischen Dünnfilm (12) umfasst.

10. Verfahren nach irgendeinem der Ansprüche 1 bis 4, wobei der Schritt des Ausbildens eines Schutzfilms (14) auf dem metallischen Dünnfilm (12) ein Ausbilden eines anorganischen Films und eines organischen Films auf den metallischen Dünnfilm (12) umfasst.

11. Verfahren nach irgendeinem der Ansprüche 1 bis 10, ferner umfassend ein Abdecken des Scintillators (16) mit einem organischen Film (18).

12. Verfahren nach Anspruch 11, wobei ein Schritt eines Abdeckens des Scintillators (16) mit einem organischen Film (18) ein Erstrecken des organischen Films (18) hin zu mindestens einem Bereich der Oberfläche des Strahlung-transmittierenden leitenden Substrats (10) umfasst.

13. Verfahren nach Anspruch 12, wobei der Schritt des Abdeckens des Scintillators (16) mit einem organischen Film (18) ein Abdecken im Wesentlichen der ganzen Oberfläche des Substrats (12) mit dem organischen Film (18) umfasst.

14. Verfahren nach irgendeinem der Ansprüche 1 bis 13, wobei der Schritt des Ausbildens des nicht-leitenden Zwischenfilms (11) auf dem Strahlung-transmittierenden leitenden Substrat (10) ein Ausbilden von entweder einem organischen Film oder einem anorganischen Film oder einer Kombination der Beiden auf dem Strahlung-transmittierenden leitenden Substrat (10) umfasst.

15. Verfahren nach irgendeinem der Ansprüche 1 bis 14, wobei der metallische Dünnfilm (12) an dem nicht-leitenden Zwischenfilm (11) und dem Schutzfilm (14) angehaftet wird und durch beide versiegelt wird.

16. Verfahren nach Anspruch 15, wobei der nicht-leitende Zwischenfilm (11) im Wesentlichen das Strahlung-transmittierende leitende Substrat (10) umhüllt.

17. Verfahren nach irgendeinem der Ansprüche 11, 12 oder 13, wobei der Schritt des Ausbildens des organischen Films (18) auf dem Scintillator ein Ausbilden des organischen Films (18) auf dem Scintillator (16) verwendend chemische Gasphasenabscheidung (CVD) umfasst.

18. Verfahren nach irgendeinem der Ansprüche 15 bis 17, wobei der nicht-leitende Zwischenfilm (11) ein Xylylene-basierter Film ist.

19. Verfahren nach Anspruch 1, wobei der Schritt (a) des Ausbildens des nicht-leitenden Zwischenfilms (11) auf dem Strahlung-transmittierenden leitenden Substrat (10) ein Ausbilden eines Zwischenfilms, der mindestens eines aus Polyimid, Epoxid-basiertem Harz, Acryl-basiertem Harz, SiO₂ oder SiN beinhaltet, auf einem Al-, Be- oder amorphen Kohlenstoffsubstrat um-fasst;
wobei der Schritt (b) des Ausbildens des metallischen Dünnfilms (12) auf dem nicht-leitenden Zwischenfilm (11) ein Ausbilden eines metallischen Dünnfilms (12), der eine Substanz beinhaltet, die aus der Gruppe ausgewählt ist, die aus Al, Ag, Cr, Cu, Ni, Ti, Mg, Rh, Pt und Au besteht, auf dem nicht-leitenden Zwischenfilm (11) umfasst;
und wobei ferner der Schritt (c) des Ausbildens des Schutzfilms (14) auf dem metallischen Dünnfilm (12) ein Ausbilden von Polyimid auf dem metallischen Dünnfilm (12) umfasst.

20. Verfahren nach irgendeinem der vorhergehenden Ansprüche, ferner umfassend ein Anordnen eines Licht-empfangenden Abschnitts eines Bil-derfassungselements (20) zur Ausrichtung auf eine Endseite des Scintillators (16), um einen Strahlungsbildsensor auszubilden.

## Revendications

1. Procédé de fabrication d'un panneau de scintillateur (1), comprenant les étapes consistant :
(a) à former un film intermédiaire non conducteur (11) sur un substrat conducteur transmettant les rayonnements (10) ;
(b) à former un film métallique mince (12) sur le film intermédiaire non conducteur (11), le film métallique mince (12) pouvant transmettre un rayonnement et réfléchissant la lumière d'une longueur d'onde prédéterminée ;
(c) à former un film protecteur (14) sur le film métallique mince (12), et
(d) à déposer un scintillateur (16) sur le film protecteur (14), le scintillateur comprenant un grand nombre de cristaux en colonnes pour convertir un rayonnement en lumière de longueurs d'onde qui peut être réfléchie par le film métallique mince (12) ; ce qui permet au film protecteur (14) d'empêcher le contact entre le film métallique mince (12) et le scintillateur (16), et ce qui permet en outre au film intermédiaire non conducteur (11) de se coller au substrat conducteur transmettant les rayonnements (10) et au film métallique mince (12), et d'empêcher le contact entre ledit substrat conducteur transmettant les rayonnements (10) et ledit film métallique mince (12).

2. Procédé selon la revendication 1, dans lequel ladite étape (a) de formation du film intermédiaire non conducteur (11) sur ledit substrat conducteur transmettant les rayonnements (10) comprend le fait de former le film intermédiaire non conducteur (11) soit sur un substrat en aluminium soit sur un substrat ayant le carbone comme composant principal.

3. Procédé selon la revendication 1 ou 2, dans lequel ladite étape (a) de formation dudit film intermédiaire non conducteur (11) sur ledit substrat conducteur (10) comprend le fait de former le film intermédiaire non conducteur (11) sur ledit substrat conducteur (10) qui contient du carbone amorphe.

4. Procédé selon l'une quelconque des revendications 1 à 3, dans lequel ladite étape (b) de formation d'un film métallique mince (12) sur le film intermédiaire non conducteur (11) comprend le fait de former un film métallique mince (12) réalisé en un matériau contenant une substance choisie dans le groupe consistant en Al, Ag, Cr, Cu, Ni, Ti, Mg, Rh, Pt et Au, sur le film intermédiaire non conducteur (11).

5. Procédé selon l'une quelconque des revendications 1 à 4, dans lequel ladite étape de formation d'un film protecteur (14) sur le film métallique mince (12) comprend le fait de former un film inorganique sur le film métallique mince (12).

6. Procédé selon la revendication 5, dans lequel ladite étape de formation d'un film inorganique sur le film métallique mince (12) comprend le fait de former un film inorganique, réalisé en un matériau contenant une substance choisie dans le groupe consistant en LiF, MfF₂, SiO₂, TiO₂, Al₂O₃, MgO et SiN sur le film métallique mince (12).

7. Procédé selon la revendication 5, dans lequel ladite étape de formation d'un film inorganique sur le film métallique mince (12) comprend le fait de former un film inorganique réalisé en un matériau contenant une substance choisie dans le groupe consistant en Al, Ag, Cr, Cu, Ni, Ti, Mg, Rh et Pt sur le film métallique mince (12).

8. Procédé selon l'une quelconque des revendications 1 à 4, dans lequel ladite étape (c) de formation du film protecteur (14) sur le film métallique mince (12) comprend le fait de former un film organique sur le film métallique mince (12).

9. Procédé selon la revendication 8, dans lequel ladite étape de formation d'un film organique sur le film métallique mince (12) comprend le fait de former un film organique réalisé en un matériau contenant une substance choisie dans le groupe consistant en matériaux à base de polyimide et de xylylène, sur le film métallique mince (12).

10. Procédé selon l'une quelconque des revendications 1 à 4, dans lequel ladite étape de formation d'un film protecteur (14) sur le film métallique mince (12) comprenant le fait de former un film inorganique et un film organique sur le film métallique mince (12).

11. Procédé selon l'une quelconque des revendications 1 à 10, comprenant en outre le fait de couvrir ledit scintillateur (16) avec un film organique (18).

12. Procédé selon la revendication 11, dans lequel l'étape de couverture du scintillateur (16) avec un film organique (18) comprend le fait d'étendre le film organique (18) jusqu'à au moins une partie de la surface dudit substrat conducteur transmettant les rayonnements (10).

13. Procédé selon la revendication 12, dans lequel ladite étape de couverture du scintillateur (16) avec un film organique (18) comprend le fait de couvrir sensiblement toute la surface dudit substrat (12) avec le film organique (18).

14. Procédé selon l'une quelconque des revendications 1 à 13, dans lequel ladite étape de formation du film intermédiaire non conducteur (11) sur le substrat conducteur transmettant les rayonnements (10) comprend le fait de former soit un film organique soit un film inorganique, soit une combinaison des deux, sur le substrat conducteur transmettant les rayonnements (10).

15. Procédé selon l'une quelconque des revendications 1 à 14, dans lequel ledit film métallique mince (12) est collé audit film intermédiaire non conducteur (11) et audit film protecteur (14) et est scellé par les deux.

16. Procédé selon la revendication 15, dans lequel ledit film intermédiaire non conducteur (11) enveloppe sensiblement ledit substrat conducteur transmettant les rayonnements (10).

17. Procédé selon l'une quelconque des revendications 11, 12 ou 13, dans lequel l'étape de formation du film organique (18) sur le scintillateur comprend le fait de former le film organique (18) sur le scintillateur (16) en utilisant un dépôt chimique en phase vapeur (CVD).

18. Procédé selon l'une quelconque des revendications 15 à 17, dans lequel ledit film intermédiaire non conducteur (11) est un film à base de xylylène.

19. Procédé selon la revendication 1, dans lequel l'étape (a) de formation du film intermédiaire non conducteur (11) sur le substrat conducteur transmettant les rayonnements (10) comprend le fait de former un film intermédiaire contenant au moins l'un(e) parmi le polyimide, une résine à base d'époxy, une résine à base d'acrylique, le SiO₂, et le SiN, sur un substrat en Al, en Be ou en carbone amorphe ;
dans lequel l'étape (b) de formation du film métallique mince (12) sur le film intermédiaire non conducteur (11) comprend le fait de former un film métallique mince (12) contenant une substance choisie dans le groupe consistant en Al, Ag, Cr, Cu, Ni, Ti, Mg, Rh, Pt et Au sur le film intermédiaire non conducteur (11) ;
et en outre dans lequel l'étape (c) de formation du film protecteur (14) sur le film métallique mince (12) comprend le fait de former un polyimide sur le film métallique mince (12).

20. Procédé de l'une des revendications précédentes, comprenant en outre le fait de disposer une partie de réception de lumière d'un élément de détection d'image (20) de manière à faire face à un côté d'extrémité du scintillateur (16), de façon à former un capteur d'image radiologique.
